# FASCICULE DE BREVET EUROPEEN

(11) **EP 0 323 861 B1**
(45) Date de publication et mention de la délivrance du brevet: **06.10.1993**
(21) Numéro de dépôt: 89102888.8
(22) Date de dépôt: 30.11.1983
(51) Int. Cl.: C12N 15/43, C12P 21/02, C07K 13/00, C07H 21/04, A61K 39/13, C12Q 1/68, G01N 33/53

(54) **Peptides comportant un site immunogène du poliovirus de la souche sabin**
Peptide, die eine Immunogen-Lage des Poliovirus des Sabin-Stammes enthalten
Peptides containing an immunogenic site of the polio virus of the Sabin strain

(30) Priorité: 30.11.1982 FR 8220115; 29.06.1983 FR 8310778
(43) Date de publication de la demande: 12.07.1989
(62) Demande divisionnaire de: 83402310.3
(73) Titulaire: INSTITUT PASTEUR, 75724 Paris Cédex 15 (FR)
(72) Inventeur: Girard, Marc, F-75015 Paris (FR); van der Werf, Sylvie, F-75004 Paris (FR)
(74) Mandataire: Gutmann, Ernest

(56) Documents cités:
- EP-A- 0 086 707
- WO-A-82/03632
- WO-A-82/04067

## Description

L'invention est relative à des peptides comportant un site immunogène du poliovirus et des fragments d'ADN contenant des séquences nucléotidiques codant pour ces peptides. L'invention concerne encore les principes vaccinants mettant en jeu de tels peptides, ces principes étant aptes à induire chez l'hôte, homme ou animal, la production d'anticorps actifs non seulement contre eux-mêmes, mais encore contre les poliovirus infectieux entiers.

On a déjà décrit dans la demande de brevet français n° 82 02013 déposée le 8 février 1982 (FR-A-2521165) des fragments d'ADN codant pour un peptide immunogène susceptible d'induire *in vivo* la synthèse d'anticorps antipoliovirus. Ces fragments d'ADN possèdent une longueur n'excédant pas celle d'un fragment d'ADN comportant de l'ordre de 1,2 ko (kilo-paire de bases). Ces fragments sont plus particulièrement caractérisés en ce qu'ils contiennent une séquence nucléotidique codant pour la protéine VP-1, laquelle s'est avérée porter des déterminants antigéniques essentiels intervenant au niveau de l'immunogénicité du poliovirus infectieux correspondant. En effet ce peptide est susceptible de former des complexes antigène-anticorps avec des sérums neutralisants monoclonaux ou polyclonaux obtenus à partir d'animaux auxquels avait été injecté du poliovirus entier (sérum de spécificité D).

Des séquences types d'ADN codant pour des peptides immunogènes du type sus-indiqué sont illustrées dans la succession des figures 1 et 2 ci-annexées, pour l'un d'entre eux,et dans la succession des figures 3 et 4, également ci-annexées,pour un autre fragment d'ADN contenant la susdite séquence. Les emplacement de certains sites de restriction auxquels il sera fait référence dans ce qui suit sont également indiqués dans ces figures. Les numérotations des nucléotides successifs intervenant dans la constitution de ces ADN s'effectuent à partir de l'extrémité 5′. Pour ce qui est de la constitution de l'ADN clonable du poliovirus dont sont issus les susdits ADN on se référera à l'article de Sylvie VAN DER WERF et autres auteurs, intitulé "Molecular cloning of the genome of poliovirus" dans Proc. Nat. Acad. Sci. USA, vol. 78, n° 10, pp. 59-83, 59-87, oct. 1981.

L'invention découle de la découverte que des peptides correspondant à des séquences d'ADN contenues dans les précédentes, mais beaucoup plus petites que celles-ci, portaient néanmoins des déterminants antigéniques permettant leur utilisation dans la constitution de principes vaccinants efficaces contre les poliovirus correspondants. Parmi les peptides en question, on peut en isoler certains dont la taille est suffisamment réduite pour qu'ils soient directement accessibles par synthèse chimique.

L'invention fournit en outre la technique permettant la détermination,au sein des ADN de tailles relativement importantes qui font l'objet de la demande de brevet français n° 82 02013,de celles des séquences d'ADN beaucoup plus petites auxquelles correspondent des peptides présentant des déterminants ou sites antigéniques les rendant aptes à être mis en oeuvre dans la production de principes vaccinants à l'égard de poliovirus correspondants entiers et infectieux.

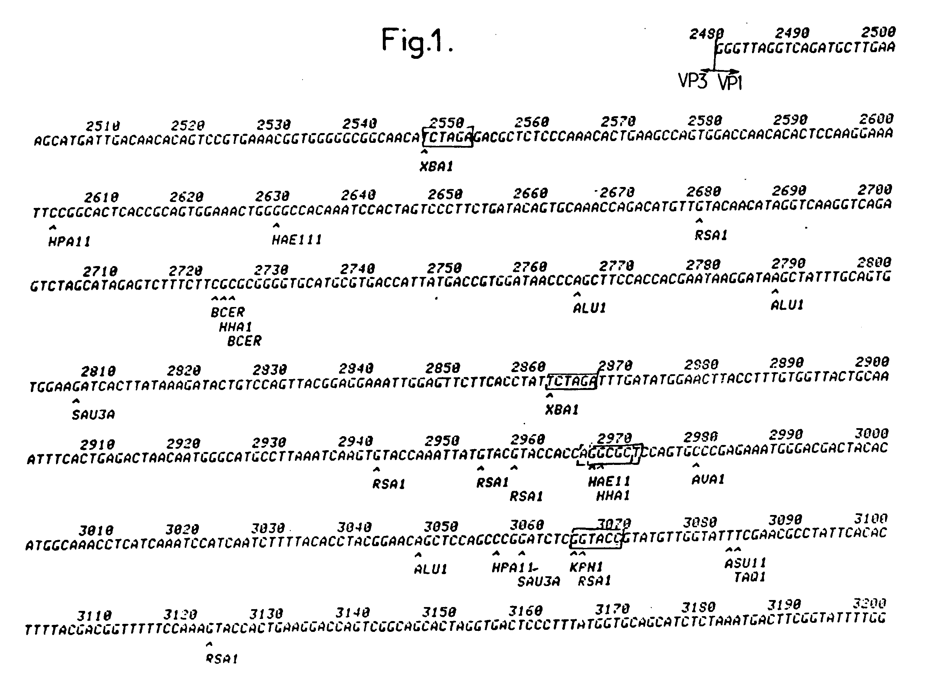

A cet égard, la plus longue des séquences d'ADN selon l'invention est constituée par le fragment délimité à ses extrémités opposées par les sites XbaI situés dans les régions définies par les positions 2546 et 2861 de la fig. 1.

L'invention concerne plus particulièrement encore celles des séquences d'ADN contenues au sein de la précédente et qui codent un peptide susceptible d'être reconnu par des anticorps monoclonaux actifs à la fois contre les particules "C" et "D" originaires d'un même poliovirus et contre le polypeptide structural VP-1 de la capside du même poliovirus. C'est de ce type d'anticorps monoclonaux qu'il s'agira en toutes circonstances dans l'exposé qui suit, sauf lorsqu'il sera spécifié autrement.

De tels anticorps sont obtenus à partir d'hybridomes, lesquels ont été obtenus par la réalisation d'une fusion entre des cellules spléniques d'un animal préalablement immunisé par un virus ou virion présentant une antigénicité "C" (obtenu notamment par chauffage pendant 1 heure à 56°C du poliovirus infectieux correspondant à antigénicité "D") et des cellules myélomateuses appropriées, en mettant en oeuvre une technique en soi connue, par la culture des hybrides ou clones cellulaires obtenus et par la sélection des clones qui s'avèrent produire des anticorps monoclonaux actifs à la fois contre les virions à antigénicité "C", les virions homologues à antigénicité "D" et contre la protéine correspondant VP-1. Les poliovirus dont il est question sont avantageusement du type 1 (Mahoney). De tels anticorps monoclonaux (qui seront encore désignés dans ce qui suit sous l'expression "anticorps CD-VP-1" (ou "C3")), les hybrides cellulaires susceptibles de les produite et un procédé d'obtention de ces derniers ont été décrits dans la demande de brevet français n° 82 19338 (FR-A 2536414) déposée le 18 novembre 1982. Deux des hybrides cellulaires formés ont été déposés dans la Collection Nationale de Culture de Micro-Organismes de l'INSTITUT PASTEUR de Paris (C.N.C.M.), respectivement sous les n° I-208 et n° I-209.

Cette séquence d'ADN selon l'invention présente la structure suivant : 
TCT AGA GAC GCT CTC CCA AAC ACT GAA
GCC AGT GGA CCA ACA CAC TCC AAG GAA ATT
CCG GCA CTC ACC GCA GTG GAA ACT GGG GCC
ACA AAT CCA CTA GTC CCT TCT GAT ACA GTG
CAA ACC AGA CAT GTT GTA CAA CAT AGG TCA
AGG TCA GAG TCT AGC ATA GAG TCT TTC TTC
GCG CGG GGT GCA TGC GTG ACC ATT ATG ACC
GTG GAT AAC CCA GCT TCC ACC ACG AAT AAG
GAT AAG CTA TTT GCA GTG TGG AAG ATC ACT
TAT AAA GAT ACT GTC CAG TTA CGG AGG AAA
TTG GAG TTC TTC ACC TAT TCT
L'invention concerne naturellement toute séquence d'ADN codant pour un peptide ayant les propriétés immunogènes semblables à celles du peptide codé par la susdite séquence nucléotidique. En particulier tout triplet de cette séquence peut être remplacé, soit par un triplet distinct codant pour le même acide aminé ou pour un acide aminé distinct, dans la mesure où la substitution du second au premier dans le peptide codé par la séquence d'ADN en question n'altérera pas fondamentalement les propriétés immunogènes du peptide codé par la séquence d'ADN ainsi modifiée. En particulier, l'invention concerne toute séquence d'ADN de ce type codant pour un peptide susceptible d'être reconnu par l'anticorps C3 sus-indiqué.

L'invention concerne encore toute séquence nucléotidique de plus faible longueur contenue dans la précédente, dès lors qu'elle code pour un peptide toujours encore susceptible d'être reconnu par l'anticorps C3.

Parmi les séquences d'ADN entrant dans le champ de l'invention, figurent celles qui contiennent les séquences nucléotidiques codant pour la séquence peptidique His 65-Phe 105 définie ci-après et plus particulièrement encore la séquence nucléotidique 2671-2792 du gène codant pour le polypeptide de structure VP-1 du poliovirus de la fig.1.

D'autres séquences préférées d'ADN entrant dans le champ de l'invention sont celles qui codent pour les séquences peptidiques His 65 -Ile 110 définies ci-après, et plus particulièrement encore la séquence nucléotidique Pro 95 -Ile 110 du même gène.

L'invention concerne naturellement les polypeptides contenant les séquences peptidiques codées par les susdites séquences d'ADN. Elle concerne en particulier la séquence de formule : 
Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser
L'invention concerne également tout peptide présentant des propriétés immunogènes équivalentes dans les conditions qui ont déjà été indiquées en rapport avec les peptides codés par les séquences d'ADN ci-dessus définies. A ce titre l'invention concerne plus particulièrement la séquence suivante, ci-après dite "séquence His 65-Phe 105". 
His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe
100
ou dite ci-après "séquence His 65 - Ile 110". 
His Val Val Gln His

Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe Ala Val Trp Lys
100
Ile
110
L'invention concerne plus particulièrement encore ceux des peptides qui contiennent la séquence peptidique suivant, ci-après dénommée Asp 93-Leu 104 : Asp Asn Pro Ala Ser Thr Thr Asn Lys Asp Lys Leu.

L'invention concerne naturellement aussi les vecteurs, notamment du type plasmides ou phages, contenant un insérat formé par l'une quelconque des séquences d'ADN, telles qu'elles ont été définies ci-dessus. Ces vecteurs modifiés peuvent être mis en oeuvre dans la transformation d'organismes cellulaires ou de micro-organismes appropriés, en vue d'induire la production par celle-ci de polypeptide, le cas échéant hybrides, contenant une séquence peptidique susceptible d'être reconnue par les anticorps monoclonaux CD-PV1 ou C3 ou d'autres anticorps reconnaissant le virus infectieux. Ces polypeptides, le cas échéant hybrides, font également partie de l'invention.

L'invention fournit encore un procédé permettant l'identification, au sein d'un séquence d'ADN normalement contenue à l'intérieur de l'ADN d'un poliovirus déterminé, de celles des séquences plus petites qui sont susceptibles de coder pour un peptide immunogène ou susceptibles d'être mises en oeuvre dans la fabrication d'un principe immunogène permettant la production d'anticorps actifs contre le poliovirus entier correspondant.

Ce procédé est essentiellement caractérisé en ce que, partant d'un plasmide contenant un insérat formé par une séquence initiale reconnue comme devant contenir une séquence plus petite susceptible de coder pour un peptide immunogène ou susceptible d'entrer dans la constitution d'un principe immunogène, on linéarise ledit plasmide au niveau d'un site de restriction extérieur à ladite séquence plus petite, on émonde de façon contrôlée le plasmide linéarisé avec une enzyme exonucléolytique, telle que l'enzyme Bal 31, on recircularise le plasmide au moyen d'un ADN-ligase, on transforme un micro-organisme approprié, lui-même transformable par le vecteur correspondant et capable d'exprimer l'insérat contenu dans celui-ci, et on détecte parmi les produits d'expression de ce micro-organisme l'éventuelle présence d'un peptide susceptible de porter le site immunogène du genre en question, par mise en contact desdits produits d'expression avec un anticorps monoclonal CD-PV1, le cycle d'opérations qui vient d'être défini étant répété jusqu'à la disparition de la détection dudit peptide immunogène parmi les produits d'expression dudit micro-organisme transformé par le dernier plasmide recircularisé.

Il est possible, au terme de chacun des cycles du procédé sus-défini, par exemple par comparaison des cartes de restriction du plasmide avant et après la susdite opération d'émondage, de déterminer celles des séquences d'ADN qui ont été éliminées entre deux émondages successifs et, par conséquent, lorsque cesse la possibilité de détection d'un peptide immunogène dans les conditions sus-indiquées, de corréler ce résultat à l'une des séquences supprimée au cours de l'opération d'émondage précédente,cette séquence supprimée d'ADN codant pour ledit peptide immunogène. La structure de la séquence supprimée (ou des séquences supprimées) peut naturellement découler des déterminations de séquences nucléotidiques d'extrémités effectuées, avant et après l'émondage considéré.

Un tel principe sera illustré dans l'un des exemples de mise en oeuvre de l'invention dont la description suit. Il sera également fait référence dans ce qui suit aux dessins dans lesquels :

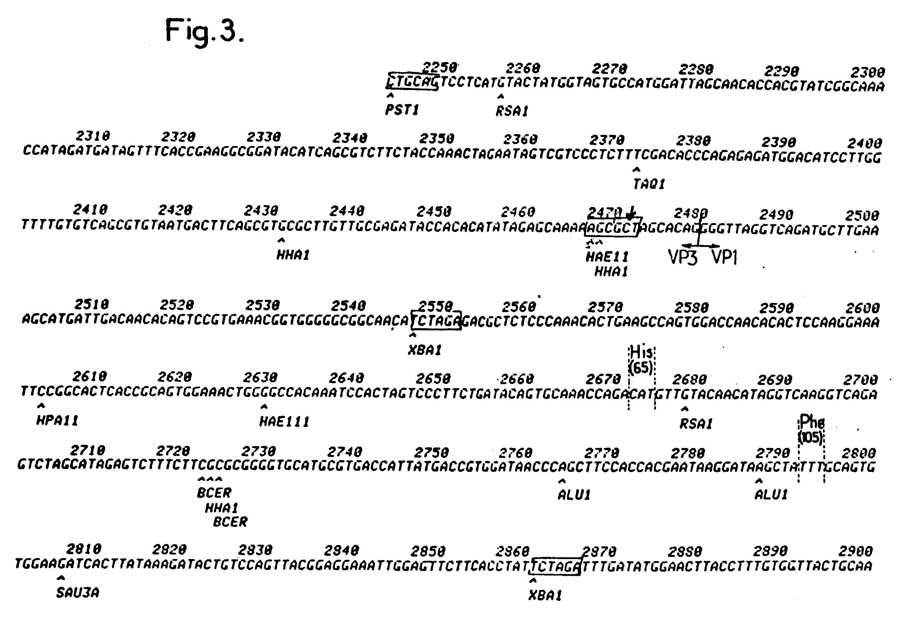

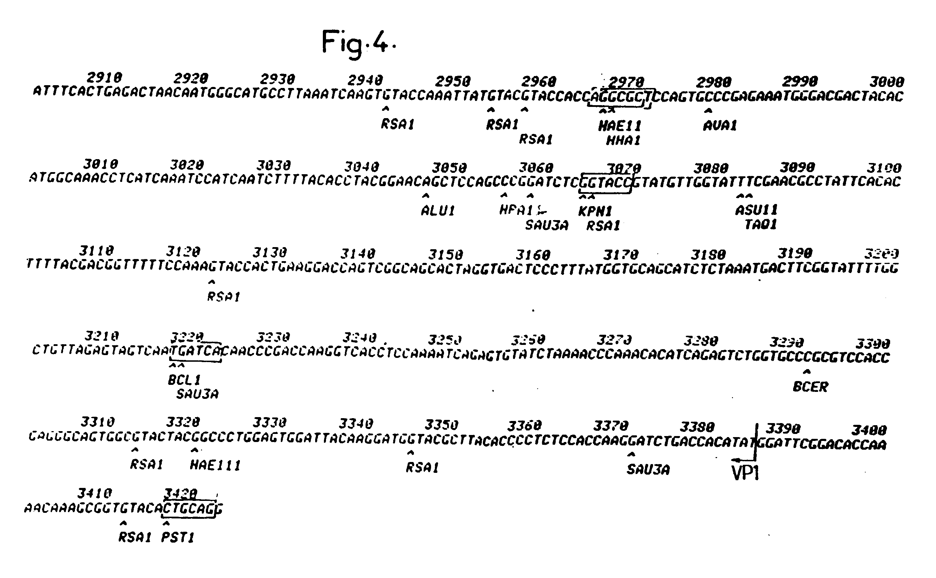

- les fig. 1 à 4 correspondent à des séquences déjà définies dans ce qui précède;

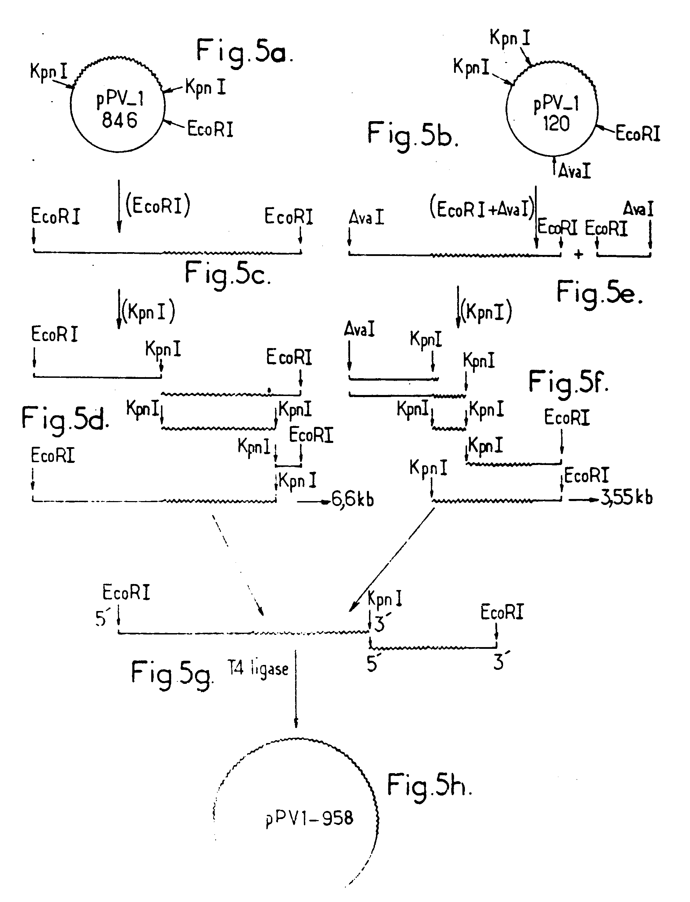

- les fig. 5a à 5h schématisent un mode de production d'un précurseur obtenu à partir des clones pPV1-846 et pPV1-120 décrits dans l'article de Sylvie VAN DER WERF et autres auteurs déjà mentionné plus haut;

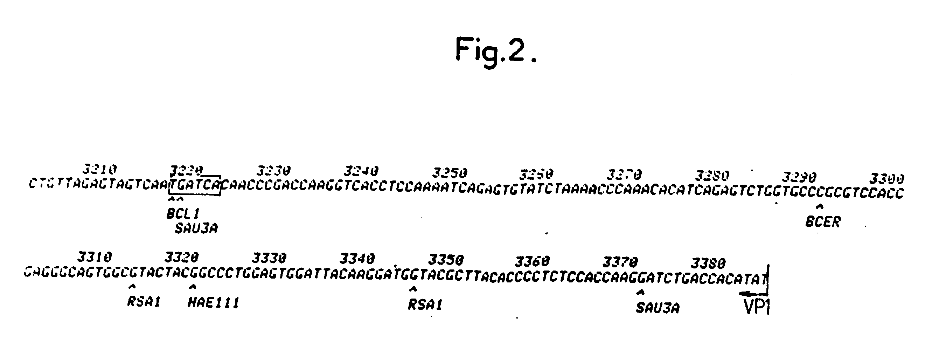

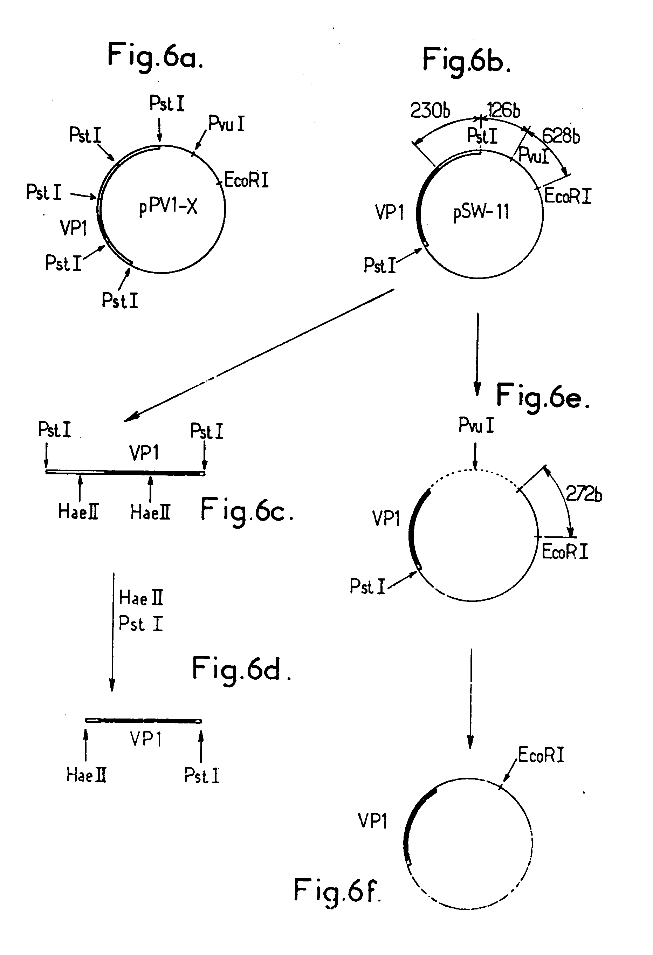

- les fig. 6a à 6f représentent schématiquement les étapes d'un mode de production d'un plasmide contenant l'essentiel de l'information génétique de la séquence d'ADN, telle qu'elle résulte des fig. 1 et 2;

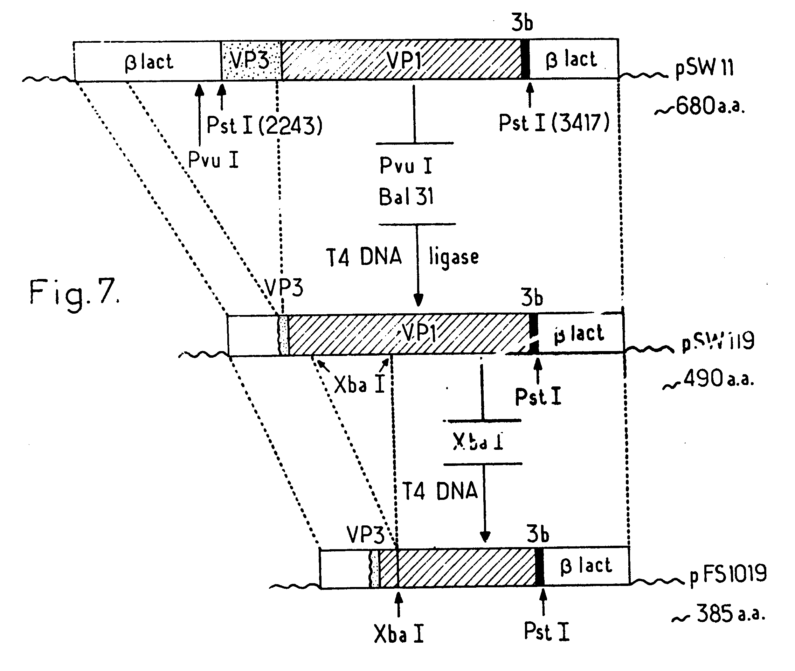

- la fig. 7 est une représentation schématique de la production du plasmide précédent et d'une étape supplémentaire mise en jeu dans une première étape de la présente invention, comme il résultera de la description qui suit.

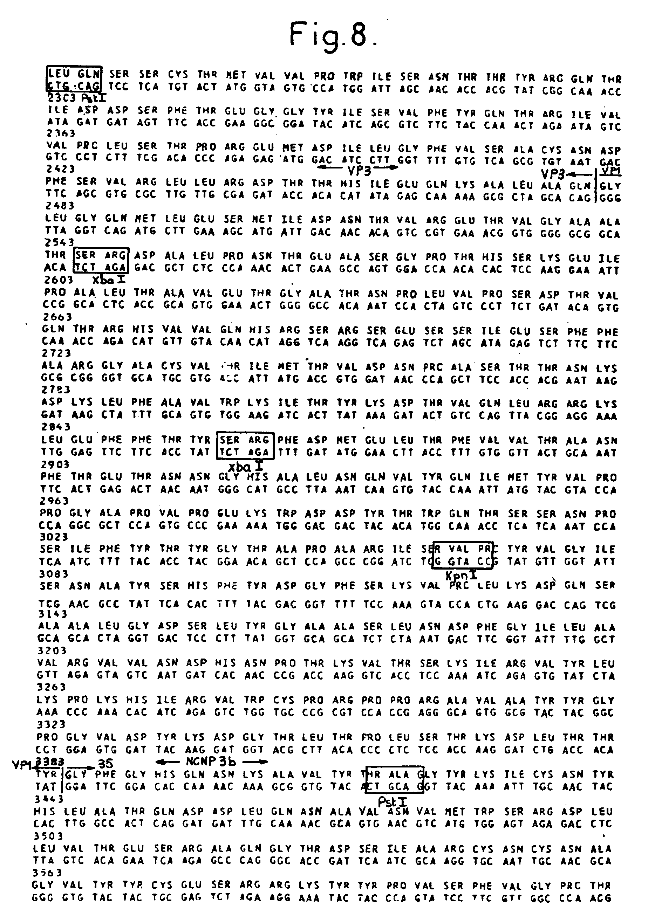

- La figure 8 est une nouvelle reproduction de la séquence codant pour VP1, précédée d'une partie de la séquence codant pour VP3 et suivie par une partie de la séquence codant pour NCVP3b. Cette séquence ne se différencie essentiellement des parties de séquences correspondantes apparaissant aux figures 1 à 4 que par la numérotation des nucléotides. Cette numérotation est conforme à celle résultant du "consensus" auquel se réfèrent A.J. DORNER et al dans l'article intitulé : "Identification of the initiation site of poliovirus polyprotein synthesis" (Identification du site d'initiation de la synthèse de la polyprotéine de poliovirus) (Journal of Virology, Juin 1982, vol.42, N° 3, pp. 1 017 - 1 028).
   Cette publication renvoie au projet MOLGEN du système SUMEX AIM de l'Université de Stanford en ce qui concerne les relations à établir entre la numérotation des séquences entièrement publiées et la numérotation adoptée dans la figure 8.

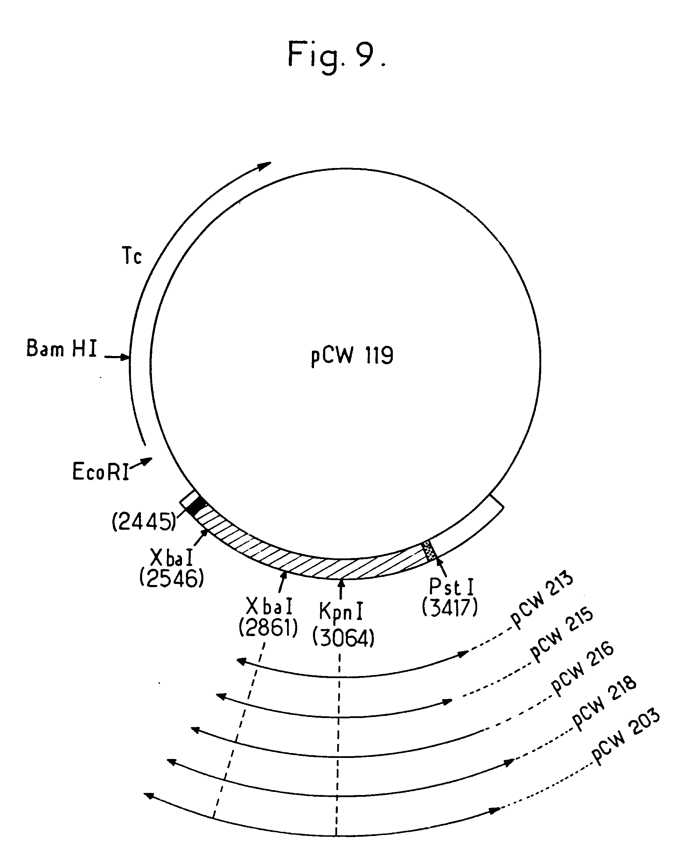

- La figure 9 est une représentation schématique du plasmide pCW 119 . Elle illustre les positions relatives des délétions introduites dans d'autres plasmides dont question plus loin et dérivés de pCW 119.

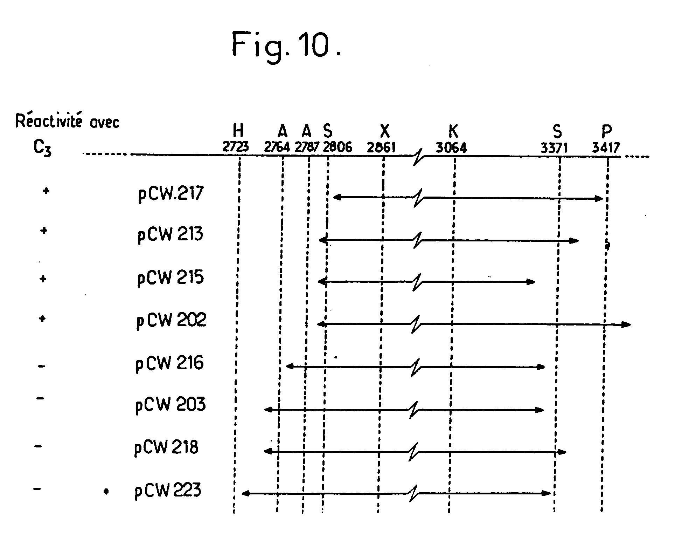

- La figure 10 illustre plus spécifiquement encore les positions de ces délétions vis-à-vis des sites de restriction déterminés dans le plasmide pCW 119.

Les techniques des constructions utilisées pour les différents plasmides sont classiques. Les ADN de plasmides ont chaque fois été clivés par les enzymes de restriction dans les conditions prévues par leurs fabricants respectifs. Les fragments d'ADN ont été analysés par électrophorèse dans un gel d'Agarose ou de polyacrylamide. Les extrémités saillantes 3′ ont été transformés en extrémités franches par incubation des fragments d'ADN (0,1 mg/ml) avec 100 µ/ml de polymérase ADN I(fragment Klenow)de E. coli pendant 1 heure à 37°C dans un milieu 10 nM Tris-HCl, ph 7,5 contenant 10 mM MgCl₂, 50 mM NaCl, 1 mM DTT en présence de 0.2 mM des premières paires de nucléotides. La digestion avec la nucléase Ba 31 a été réalisée dans un milieu 20 mM CaCl₂, 12 mM MgCl₂, en mettant en oeuvre un rapport enzyme/DNA de 0,12 u par µg. Après incubation pendant 15 minutes à 30°C, on ajoute de l'EDTA jusqu'à atteindre une concentration de 50 mM et le DNA est extrait avec du phénol et précipité avec de l'éthanol. Les réactions de ligation ont été réalisées dans 20 µl d'un milieu 60 mM Tris-HCl, ph 7,5 , 10 mM MgCl₂, 10 mM DTT, 1 mM ATP pendant 18 heures à 15°C, en utilisant 1 u de T4 DNA Ligase par µg de DNA. Les plasmides linéarisés ont, le cas échéant, été traités pendant 30 min. à 68°C avec une phosphatase alkaline bactérienne (0,02 u par µg DNA) avant ligation avec les fragments appropriés.

### 1. Hydrolyse des DNA clonés par des enzymes de restriction

1.1. L'ADN du plasmide pPVI-846 est hydrolysé complètement par EcoRI. La forme linéaire du DNA plasmidique ainsi obtenue (figure 5c) est hydrolysée par digestion partielle avec Kpn I; les fragments obtenus (figure 5d) sont séparés par électrophorèse en gel d'agarose à 0,7 %.

Le fragment de taille 6,6 kbp est sélectionné. Il représente en effet la séquence du plasmide pBR322 du site EcoRI au site Pst I, prolongée de celle du DNA correspondant à la séquence du poliovirus qu s'étend du nucléotide 1 au nucléotide 3064 (2e site Kpn I).

1.2. L'ADN du clone pPVI-120 est hydrolysé dans une digestion complète par AvaI et EcoRI formant ainsi 2 fragments de tailles différentes (figure 5e). L'ADN est hydrolysé ensuite partiellement par Kpn I. Les fragments ainsi obtenus (figure 5f) sont séparés par électrophorèse en gel d'agarose à 0,7 %.

Le fragment de taille 3,55 kbp est sélectionné. Il représente en effet la séquence du cDNA du poliovirus allant du nucléotide 3064 (2e site Kpn I) au nucléotide 5650 environ, prolongée de celle des 752 paires de bases du segment Pst I-EcoRI du plasmide pBR322.

### 2. Extraction des fragments de DNA des gels

2.1. Les fragments sont visualisés dans les gels par coloration au bromure d'éthidium; ceux de la taille voulue sont extraits des gels par électroélution en sac à dialyse.

2.2. Le matériel ainsi obtenu est purifié et concentré.

### 3. Recollage des fragments (recombinaison)

Les deux fragments sélectionnés provenant des clones pPVI-846 et pPVI-120 et décrits ci-dessus sont mélangés et recollés à l'aide de la DNA ligase du phage T4. Les bouts collants formés aux points de coupure par EcoRI et KpnI et portés à chaque extrémité des deux fragments favorisent leur recollage et assurent que celui-ci ne puisse se faire que dans le sens désiré (figures 5g et 5h).

Le génome du plasmide pBR322 est ainsi reconstitué sand modification ni délétion dans le plasmide recombinant. En particulier, les régions nécessaires à sa réplication et à l'expression de la résistance à la tétracycline ne sont pas touchées.

### 4. Transformation de la souche E.coli 1106

Les fragments des plasmides pPVI-846 et -120 recollés par leurs sites Kpn I et EcoRI sont mis en contact avec des bactéries compétentes de souche *E. coli* 1106 dans les conditions de transformation. Les colonies de bactéries résistances à la tétracycline et sensibles à l'ampicilline sont sélectionnées.

### 5. Analyse des nouveaux clones

5.1 Le DNA plasmidique des bactéries tétracycline résistantes est purifié. Sa masse est déterminée par électrophorèse en gel d'agarose. Elle est égale à celle du plasmide pBR322 augmentée des 5650 paires de bases du cDNA viral formé par recombinaison.

5.2 L'hybridation in vitro des cDNA ainsi obtenus avec des sondes spécifiques provenant des clones pPVI-846 et pPVI-120 permet la vérification de la présence dans un seul clone recombinant du matériel génétique du poliovirus inséré originellement dans les deux clones parentaux.

5.3 L'analyse détaillée des nouveaux clones est effectuée par les méthodes utilisées antérieurement pour l'étude des clones déjà caractérisés (cartographie physique par enzymes de restriction, microscopie electronique, séquence nucléotidique, etc.).

5.4 Le cDNA porté par le plasmide recombinant (pPV1-X) ou pPV1-958 porte l'information génétique nécessaire à la synthèse de la protéine NCVP1a (ou P1), précurseur des protéines de capside VP4 (nucléotides 743 à 950), VP2 (nucléotides 951 à 1766), VP3 (1767 à 2479) et VP1 (2480 à 3385), suivie de celles qui correspondant à la protéine NCVP3b (ou P2) (précursur notamment de la protéine HCVPX) et au début de la protéine NCVP1b (ou P3) L'ensemble couvre environ 5650 des 7440 bases du génome viral.

Le plasmide pPVI-846 a été déposé à la C.N.C.M. sous le n° I-155 et le plasmide 120 sous le n° I-156 le 19 mai 1981.

Le plasmide pPV1-958 obtenue contient dans son insérat la séquence nucléotidique qui code pour les protéines VPO (nucléotides 743 à 1766), VP3 (nucléotides 1767 à 2479) et VPI (nucléotides 2480 à 3385) suivie de la séquence codant pour la protéine NCVP3b (nucléotides 3386 à 5100 et quelque) et du début de celle de la protéine NCVP1b.

Partant du plasmide pPV1-958, on peut alors obtenir un fragment de cDNA codant pour VP1 en procédant comme suit.

### ISOLEMENT ET RECLONAGE D'UN FRAGMENT DE cDNA RENFERMANT LA SEQUENCE DE VP1.

La séquence de nucléotides qui code pour la protéine VP1 est encadrée, dans le génome viral, et par conséquent aussi dans l'insérat porté par pPV1-958, de deux sites PstI, situés respectivement 237 nucléotides en amont (position 2243) et 32 nucléotides en aval (position 3417) du premier et du dernier nucléotide de cette séquence (cf. carte de restriction dans la susdite publication et fig. 1 et 2).

Le découpage de pPV1-958 (fig. 6a) par l'enzyme de restriction PstI engendre donc une famille de fragments ayant des longueurs correspondant respectivement à 4,36 kb (corps du plasmide) et à 1,8 kb ; O,43 kb ; 1,17 kb et environ 2,23 kb. Le fragment de 1,17 kb porte la séquence nucléotidique codant pour la fin de VP3 et la totalité de VPI. Ce dernier fragment commence par la séquence nucléotidique : _{5′} G T C C T C A T G T A et s'achève par la séquence G T A C A C T G C A_{3'}. On le sépare des autres fragments PstI par électrophorèse en gel d'agarose. On prélève la bande du gel qui le contient, et on la soumet à une électroélution pour en extraire le DNA. On suit l'électroélution par illumination aux ultraviolets après coloration du gel au bromure d'éthidium. Le fragment ainsi préparé correspond aux nucléotides du poliovirus 2243 à 3417. On l'insère par ligation avec de la DNA-ligase au site PstI du plasmide vecteur pBr-322 préalablement linéarisé par cette même enzyme. Les plasmides recombinants qu'on a formés ainsi sont clonés dans la souche 1106 d'*Escherichia coli* (sélection des colonies devenues résistances à la tétracycline mais demeurées sensibles à l'ampicilline après transformation par le plasmide).

L'analyse de leur ADN par cartographie aux enzymes de restriction permet d'identifier et de sélectionner les plasmides recombinants qui portent le fragment du cADN polioviral inséré dans le sens contraire aux aiguilles d'une montre par rapport à la carte de pBR-322, c'est-à-dire dans le même sens transcriptionnel que le gène de la β-lactamase (gène de la résistance à l'ampicilline). Il faut noter que l'insertion du fragment 2243-3417 au site PstI de pBR-322 interrompt la continuité de la séquence nucléotidique, et inactive donc le gène de la β-lactamase du vecteur, mais ne permet cependant pas d'assurer l'expression des protéines poliovirales car elle entraîne un changement de phase de lecture de l'insérat.

On dénomme pSW-11 (fig. 6b), le plasmide ayant ces propriétés.

### ELIMINATION DES SEQUENCES CODANT POUR LA PARTIE C TERMINALE DE VP3 : EMONDAGE DE VP1.

Le plasmide pSW-11 contient, précédant, dans le sens transcriptionnel 5 → 3′, la séquence de VP1, 237 nucléotides de cADN de poliovirus relevant de la séquence de VP3. Ces nucléotides excédentaires peuvent être enlevés d'au moins deux manières :
a) par traitement ménagé du fragment PstI (préalablement extrait de pSW-11: fig. 6c) de 1,17 kb par l'enzyme de restriction HaeII (digestion partielle au nucléotide 2467), puis sélection parélectrophorèse du fragment HaeII-PstI de 0,95 kb (fig. 6d) (nucléotides polioviraux 2467 à 3417) et reclonage de ce fragment dans les plasmides appropriés. On peut faciliter le reclongage en accrochant de façon en soi connue aux extrémités du fragment émondé des adaptateurs ("linkers") synthétiques, courtes séquences de nucléotides contenant un site de restriction déterminé obtenue par synthèse, par exemple selon la technique décrite par R.H. SCHELLER et al, Science, tome 196 (1977), pp. 177-180. Le type de "linker" choisi dépend essentiellement du site de coupure de l'enzyme de restriction utilisée dans le vecteur d'expression.
b) par linéarisation du plasmide pSW-11 par digestion complète par l'enzyme PvuI, suivie d'un traitement exonucléolytique à l'enzyme Bal 31, puis de l'addition éventuelle d'adaptateurs ("linkers") synthétiques types (Biolabs, Collaborative Research) et de la recircularisation du plasmide par la DNA ligase.

On ouvre donc les molécules. On analyse par migration en électrophorèse en gel d'agarose leurs tailles pour identifier celles qui ont perdu environ 700 paires de bases(perte qui dans la figure 6e est symbolisée par un arc de cercle en pointillés), c'est-à-dire quelque 350 de part et d'autre du site PvuI, soit le fragment PvuI-PstI de pBR-322 plus la séquence de VP3 jusqu'à VP1, d'un côté,et en une longueur similaire de pBR-322 allant de PvuI vers EcoRI, de l'autre côté.

De cette façon on peut isoler un fragment dont l'une des extrémités coïncide avec l'extrémité de la séquence d'ADN codant pour VP1 ; ou en tous les cas en est très proche.

En effet, le site PvuI se trouvait à 126 paires de bases (b) du site proximal PstI de la séquence du fragment PstI de 1,17 kb et à 363 paires de bases de l'extrémité proximale du fragment de cDNA codant pour VP1, dans le plasmide pSW-11.

Après fixation éventuelle aux extrémités du fragment sélectionné de "linkers" contenant par exemple un site BglII au moyen d'une ligase, on sélectionne les plasmides dont la taille est de 4,8 à 5 kb (fig. 6f). On détermine ensuite ceux des plasmides qui ont conservé la séquence entière de VP1, tout en ayant perdu la totalité ou la quasi-totalité de VP3. On vérifie cela dans le cas où l'on a inséré un linker BglII en déterminant la séquence nucléotidique du fragment BglII-PstI des plasmides sélectionnés. Si l'on utilise la méthode de SANGER, on insère les fragments à séquencer dans la forme réplicative du phage M13 et on clone les phages, recombinants ainsi constitués. On utilise ensuite leur ADN pour séquencer le fragment inséré, selon la technique décrite par SANGER. On peut aussi procéder à la détermination de la séquence nucléotidique conservée par la méthode décrite par MAXAM et GILBERT.

Le plasmide obtenu par émondage du plasmide pSW-11, notamment par la variante b du procédé décrit ci-dessus, mais sans introduction de linker BglII, a été dénommé pSW-119.

Les différences observées entre les plasmides pSW-11 et pSW-119 (ou pCW-119) résultent du schéma de la fig. 7. En particulier, le plasmide pSW-119 a perdu la plus grande partie de la partie de séquence que contenait encore le plasmide pSW-11 et qui code pour le polypeptide de structure VP3 du poliovirus.

Comme cela a été indiqué dans la demande de brevet français n^{o} 82 02013 le plasmide pSW-119 est capable d'exprimer une protéine de fusion VP1-β-lactamase dans des souches de *E. coli* 1106 ou GC 26 (entre autres micro-organismes, tels que ceux envisagés dans la demande de brevet antérieur n° 82 02013). Cette protéine de fusion, ayant un poids moléculaire de 49.000 daltons, est spécifiquement immunoprécipitée par les anticorps monoclonaux CD-VP1 (ou C3).

Un dérivé de pSW 119, pFS119,a été construit en remplaçant les séquences entre les sites BamHI et Pst I de pBR 322 (nucléotides 375 -3 608) par les séquences correspondantes de pBR 327. Après marquage des protéines exprimées par le plasmide pFS 119 dans des bactéries GC 26 avec la (³⁵S)méthionine, immunoprécipitation et analyse par électrophorèse sur gel de polyacrylamide, il a de nouveau été possible de reconnaître une protéine de fusion ayant un poids moléculaire de l'ordre de 49 000 daltons (p49),spécifiquement immunoprécipitée par C3,parmi les produits d'expression dans GC 26.

On a également représenté dans la fig 7 la structure schématique du plasmide pFS-1019, tel qu'il a été obtenue par :
- digestion du plasmide pSW-119 ou pFS-119
- séparation par électrophorèse des fragments obtenus en gel d'agarose,
- sélection (par la taille des fragments concernés) de celui des fragments dérivés de pSW-119 ou pCW-119 et présentant la taille de celui-ci, cependant réduite d'environ 315 paires de bases. On a de même recueilli le petit fragment de 315 paires de bases XbaI-XbaI, également obtenu à partir du milieu de digestion dans les mêmes conditions.

Le premier fragment sélectionné a été recircularisé au moyen d'une ligase, pour former le plasmide pFS 1019. Après incorporation de celui-ci dans E. coli 1106, celle-ci a conduit à l'obtention d'une protéine de fusion tronquée, de 39.000 daltons, qui n'est plus reconnue par l'anticorps monoclonal CD-VP1 ou C3.

Au contraire, le petit fragment a extrémité XhaI de 315 nucléotides, conduit, lorsqu'il est réintroduit en phase dans un gène porté par un plasmide approprié, à un plasmide modifié capable de transformer E. coli 1106 pour rendre celle-ci capable d'exprimer une protéine hybride reconnue par les anticorps monoclonaux C3. Par exemple,cette réintroduction en phase peut être effectuée dans le gène de β-lactamase de pBR-322.

La mise en phase adéquate peut, si besoin, être réalisée par la technique décrite dans la demande de brevet français n^{o} 78 32041 du 13 novembre 1978.

La réinsertion du fragment XbaI-XbaI, quel que soit son origine, dans le plasmide pFS 1019 conduit à nouveau à un plasmide dont les produits d'expression contiennent une protéine reconnaissable par C3. Il en a particulièrement été ainsi en ce qui concerne le plasmide pCW 119,lequel a été obtenue par réinsertion dans le site XbaI de pFS 1019 d'un fragment XbaI·XbaI de même taille et structure nucléotidique, isolé à partir de pPV1-366 également décrit dans la demande de brevet 81 09 968.

La séquence nucléotidique du fragment XbaI-XbaI (315 paires de nucléotides) a déjà été indiquée plus haut. De même a été indiquée plus haut la séquence peptidique du peptide Ser 23- Ser 128.

On a représenté dans la figure 9 un schéma du plasmide pCW 119, dans lequel la séquence codant pour la protéine VP 1 a été représentée par une zone hachurée délimitée par deux arcs de cercles. Les principaux sites considérés dans le cadre de la présente description sont également indiqués dans la figure 9.

La détermination et l'obtention de séquences peptidiques plus petites susceptibles de porter le site immunogène recherché (ou épitope reconnu par C3) ont été conduites de la façon suivante.

Le plasmide pCW 119 a été soumis à une digestion par l'enzyme de restriction Kpn I, ce qui l'a ouvert au niveau du site de restriction 3064 (par conséquent extérieur au fragment XbaI susdit). La mise en oeuvre du procédé défini ci-dessus, mettant en jeu des cycles répétés d'émondage avec l'enzyme Bal 31, a conduit à la perte de fragments successifs d'extrémités, dont ceux codant pour les susdites séquences "His 65-Phe 105" et "Asp 93-Leu 104". La délétion à partir des plasmides linéarisés des fragments contenant les séquences codant pour les séquences peptidiques qui viennent d'être mentionnées,s'est manifestée par la perte chez le plasmide postérieurement recircularisé (au moyen de T4-ADN-ligase) de sa capacité d'induire la production, par les bactéries transformées par lui, de séquences peptidiques susceptibles d'être reconnues par les anticorps monoclonaux CD-PV1 ou C3.

Afin de localiser avec davantage encore de précision l'épitope reconnu par C3, on a construit une série de plasmides dérivés de pCW 119 et comportant des délétions plus ou moins étendues de cette séquence. Les positions relatives des fragments délétés vis-à-vis des sites XbaI (2546)et(2 861) sont schématisées par les arcs de cercles apparaissant dans la figure 9 . Les limites de ces délétions vers la gauche ont été déterminées par linéarisation des plasmides (à raison de 1 µg d'ADN) avec XbaI et par marquage avec l'enzyme de Klenow pendant une heure à 15°C, en présence de [α ³²P]-dATP (10 µCi) et de dGTP,dCTP et dTTP (à raison de 0,2 mM de chacun de ces derniers constituants). L'ADN marqué a ensuite été digéré au moyen des enzymes de restriction indiqués ci-après (conditions de digestion partielle lorsque l'on a recours à AluI) .Les fragments de restriction marqués ont ensuite été séparés sur un ge de polyacrylamide à 5 % et rendus visibles par autoradiographie. Les limites des délétions vers la droite ont été déduites de la taille des fragments délétés et confirmées par la présence ou l'absence des sites de restriction pour les enzymes identifiés dans la partie supérieure de la fig. 10. Les symboles utilisés dans cette dernière avaient la signification suivante :
X = XbaI; H = HhaI; A=AluI; S = Sau3A; K = KpnI P = PstI. Les numéros indiqués correspondent aux positions des nucléotides concernés vis-à-vis de la fig 8.

Les protéines de fusion tronquées exprimées par les plasmides pCW217, 213, 215, et 202 réagissent encore avec l'anticorps monoclonal neutralisant C3. En revanche, les protéines de fusion tronquées exprimées par les plasmides pCW216, 203, 218 et 223 ne sont plus reconnues par l'anticorps C3. La cartographie fine par enzymes de restriction a permis de déterminer que la plus grande délétion n'affectant pas la réactivité de la protéine tronquée avec C3 (pCW215) s'étend jusqu'au nucléotide 2792 
(Leu)
104
et que la plus petite délétion se traduisant par une perte de réactivité de la protéine tronquée s'étend jusqu'aux nucléotides 2771-2782

(Thr-Lys)
 98  108

Par conséquent, il peut être considéré que l'extrémité C-terminale de la séquence d'acides aminés constituant l'épitope neutralisant reconnu par C3 est localisée entre les acides aminés 95, 110, et plus particulièrement encore les acides aminés 98 et 104 de la protéine VP1. Cette région correspond par ailleurs à une zone hydrophile de la protéine.

### INSERTION DE CES SEQUENCES D'ADN DANS UN VECTEUR D'EXPRESSION

La séquence XbaI-XbaI ne comporte ni codon d'initiation, ni codon de terminaison. Elle ne comporte non plus ni promoteur pour sa transcription, ni signal de reconnaissance par les ribosomes (séquences de SHINE et DALGARNO, décrite dans GIRARD et HIRTH, Virologie Moléculaire, Edition Doin 1980, pp. 15-46 et 263-264). Pour la faire exprimer, il faut donc l'insérer en phase au milieu de la séquence nucléotidique 'et en tout cas derrière l'AUG d'initiation) d'un gène cloné avec son promoteur (ou auquel on adjoindra en amont, un promoteur étranger). L'utilisation de "linkers", comme décrit ci-dessus, permet d'envisager l'utilisation de plusieurs types de vecteurs d'expression différents selon le promoteur considéré: par exemple du type de ceux indiqués ci-après à titre d'exemple.

### a) Promoteurs bactériens

C'est notamment le cas des plasmides contenant la région promoteur-opérateur de l'opéron lactose d'E coli (opéron lac), suivie de la partie 5′ du gène de la β -galactosidase. Ces vecteurs, du type pPC (CHARNAY et al, Nucleic Acid Research 1978, tome V, pp. 4 479-4494), permettent l'insertion de la séquence au site EcoRI situé à 21 nucléotides derrière l'AUG d'initiation de la β-galactosidase. La protéine à laquelle ils donnent naissance comporte donc pour extrémité N terminale les sept (ou huit) premiers acides aminés de la β -galactosidase bactérienne, suivis des acides aminés codés par ladite séquence.

### b) Promoteurs de phage

C'est notamment le cas des plasmides contenant la région promoteur-opérateur de l'opéron de gauche (P_{L}) ou de l'opéron de droite (P_{R}) du phageλ .Ces vecteurs, respectivement du type pKC30 (ROSENBERG, Nature 1981, vol.292, p. 128) ou pCL47 (ZABEAU et STANLEY, The EMBO Journal, 1982, tome I, pp.1217 - 1224)dérives du pLK5 (ou pRC5) et de pLG400, ce dernier étant décrit dans Cell, 1980, vol. 20, pp. 543-553) permettent d'effectuer l'insertion de ladite séquence au sein des séquences nucléotidiques codant respectivement pour l'extrémité N-terminale du produit du gène N ou pour celle du produit du gène cro déposé le 8 février 1982 à la C.N.C.M. sous le n° I-184. Ces systèmes de vecteurs sont propagés à 30°C dans des bactéries lysogénisées par un phageλ à répresseur thermosensible (cl 857) ou en présence de plasmides porteur du même gène (cl 857)codant pour un répresseur thermosensible. Ils demeurent inactifs, du fait du répresseur, tant que la culture est maintenue à 30°C. Le transfert de la culture à 42°C est suivi de l'activation des promoteurs de λ (P_{L} ou P_{R}) portés par le plasmide recombinant, du fait de l'inactivation du répresseur du gène cI 857.

### c) Promoteurs viraux

C'est en particulier le cas de l'utilisation du virus SV40 comme vecteur. Dans ce cas, on utilise le promoteur viral tardif et l'on insère la séquence du poliovirus à la place de tout ou partie de la région codant pour les protéines tardives de SV40 (VP1 ou VP2). On construit ainsi des ADN de SV40 substitués dans lesquels les séquences codant pour les protéines de capside de ce virus sont remplacées par la séquence codant pour le peptide immunogène. Ainsi l'insertion de ladite séquence, au besoin par l'intermédiare de "linkers" appropriés à la place du fragment tardif HaeII-PstI de SV40 (nucléotides de 767 à 1923), ou d'une partie de ce fragment,aboutit à la création d'un gène chimérique possédant une séquence codant pour un peptide immunogène inducteur in vivo d'anticorps actifs à l'égard du poliovirus directement en aval de la portion N-terminale de la protéine VP2 du SV40.

On peut procéder de même en substituant la séquence VP1 du poliovirus à celle du SV40 entre les sites EcoRI (1718) et Bam H1 (2469).

### d) Promoteurs de virus animaux portés par des plasmides bactériens

C'est le cas des plasmides portant les promoteurs du gène de la thymidine-kinase du virus de l'herpès (pAGO), du gène de l'antigène HBs du virus de l'hépatite B (pAC-2 ou pAC-14) ou des gènes précoces ou tardifs de l'adénovirus 2 etc.. L'insertion d'une séquence immunogène selon l'invention derrière l'AUG du gène viral cloné avec son promoteur permet d'en assurer l'expression dans la cellule animale (après transfection, micro-injection ou fusion cellules-protoplastes).

Les séquences peptidiques ou "séquences selon l'invention" sont accessibles par synthèse chimique, par exemple en ayant recours à l'un des procédés classiques dont les conditions de mise en oeuvre sont rappelées ci-après.

La synthèse des peptides en solution homogène et en phase solide est bien connue.

A cet égard, on pourra avoir recours au mode de synthèse en solution homogène décrit par HOUBENWEYL dans l'ouvrage intitulé "Methodem der Organischen Chemie" (Methode de la chimie organique) édité par E. Wünsch., vol. 15-I et II, THIEME, Stuttgart 1974.

Cette méthode de synthèse consiste à condenser successivement deux à duex les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthylaminopropyl)-carbodiimide. Lorsque l'aminoacyle mis en oeuvre possède une fonction amine supplémentaire (cas de la lysine par exemple) ou une autre fonction acide (cas par exemple de l'acide glutamique), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple, lorsque l'un des aminoacyles considérés contient une fonction SH (par exemple le cystéine), on pourra avoir recours à un groupe acétamidométhyle ou paraméthoxybenzyle.

Dans le cas de la syntnèse progressive, acide aminé par acide aminé, la synthèse débute de préférence par la condensation de l'amino-acide C-terminal avec l'amino-acide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymere très poreuse, sur laquelle on fixe le premier amino-acide C-terminal de la chaîne. Cet amino-acide est fixé sur la résine par l'intermédiaire de son groupe carboxylique et sa fonction amine est protégée, par exemple par le groupe t-butyloxycarbonyle.

Lorsque le premier amino-acide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième amino-acide qui fournit le second amino-acyle de la séquence recherchée, à partir du résidu amino-acyle D-terminal sur la fonction amine déprotégée du premier amino-acide C-terminal fixé sur la chaîne. De préférence, la fonction carboxyle de ce deuxième amino-acide est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux amino-acides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

### DETECTION DE L'EXPRESSION DES SEQUENCES IMMUNOGENES SELON L'INVENTION

L'expression des plasmides recombinants porteurs desdites séquences immunogènes et capables de les exprimer, c'est-à-dire d'effectuer la synthèse d'un peptide immunogène, est détectée par des techniques d'immunoprécipitation, en elles-mêmes connues et mettant de préférence en jeu des liquides d'ascite contenant les anticorps monoclonaux C3 ou sérum de lapin anti-VP1 (α VP1).

En ce qui concerne les séquences de plus petite taille et portant un épitope ou déterminant immunogénique, et plus particulièrement celles qui sont accessibles de façon relativement aisée par synthèse chimique, il sera souhaitable, en vue d'accentuer leur caractère immunogène in vivo, de les coupler ou "conjuguer" de façon convalente à une molécule porteuse, physiologiquement acceptable et non toxique.

A titre d'exemples de molécules porteuses ou supports macromoléculaires entrant dans la constitution des conjugués selon l'invention, on mentionnera des protéines naturelles, telles que la toxine tétanique, l'ovalbumine, des sérums albumines, des hémocyanines, etc.

A titre de supports macromoléculaires synthé tiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20.000.

Pour synthétiser les conjugués selon l'invention, on peut avoir recours à des procédés connus en soi, tel que celui décrit par FRANTZ et ROBERTSON dans Infect. and Immunity, 33, 193-198 (1981), ou celui décrit dans Applied and Environmental Microbiology, octobre 1981, vol. 42, n° 4, 611-614 par P. E. KAUFFMAN en utilisant le peptide et la molécule porteuse appropriée.

Dan la pratique, on utilisera avantageusement comme agent de couplage les composés suivants, cités à titre non limitatif : aldéhyde glutarique, chloroformiate d'éthyle, carbodiimides hydrosolubles [N-éthyl-N′(3-diméthylamino-propyl) carbodiimide, HCl], diisocyanates, bis-diazobenzidine, di- et trichloro-s-triazines, bromures de cyanogène, benzaquinone, ainsi que les agents de couplage mentionnés dans Scand. J. Immunol., 1978, vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminopropyl)-carbodiimide, le N-hydroxybenzotriazole, etc. On peut encore avoir recours à la glutaraldéhyde, notamment lorsqu'il s'agit de relier entre eux des groupes aminés respectivement portés par le peptide et la molécule support.

On mentionne ci-après à titre d'exemple le couplage du peptide Asp 93-Leu 104 à une molécule support constituée par l'hémocyanine(KLH) de l'anglais "Keyhole limpet hemocyanin" au moyen de glutaraldéhyde selon la méthode décrite par BOQUET, P. et Coll. (1982) Molec. Immunol., 19, 1541-1549. Le couplage est fait à partir de proportions d'environ 2 mg de peptide pour 2,25 mg de l'hémocyanine.

Le conjugué obtenu est immunoprécipitable par des anticorps monoclonaux C3.. Cette immunoprécipitation peut être suivie par marquage du conjugué avec ¹²⁵I en utilisant de la chloramine T. Etant donné que le peptide ne contient pas de résidus tyrosine, le marquage n'intervient qu'au niveau de la protéine support, de sorte que les propriétés antigéniques du peptide n'ont pas pu être modifiées.

L'immunogénicité de ces peptides peut également être renforcée, en produisant leur oligomérisation, par exemple en présence de glutaraldéhyde ou tout agent permettant la mise en jeu du couplage de fonctions réactives distinctes portées par chacun des peptides monomères; en particulier, l'invention concerne les oligomères immunogènes hydrosolubles ainsi obtenus, comprenant notamment de 2 à 10 motifs monomères.

D'une façon générale, l'invention concerne tous petits "peptides immunogènes" contenant moins de 20 résidus aminoacyle, de préférence moins de 15 résidus aminoacyle. Ces peptides immunogènes contiennent de préférence la séquence Asp 93-Leu 104 sus-indiquée ou toute séquence ayant une structure conformationnelle semblable.

L'invention ne se limite naturellement pas aux peptides particuliers qui ont été envisagés.

Comme il est bien connu de l'homme de l'art, certains résidus aminoacyle contenus dans les séquences considérées peuvent éventuellment être remplacés par d'autres résidus aminoacyle, dans la mesure où ceux-ci ne modifient pas sensiblement les configurations de surface des peptides formés,et leur aptitude, notamment après leur couplage avec le support macromoléculaire, à réagir avec les anticorps dirigés contre les poliovirus. A cet égard, on mentionnera par exemple les éventuelles substitutions du groupe alanyle par le groupe glycyle ou vice-versa, la substitution possible des résidus iso-asparagiques par des résidus aspartiques, glutamine ou isoglutamine, la substitution des groupes valine par les groupes alanine, leucine ou glycine, la substitution des groupes lysine par des groupes norleucine ou encore arginine, etc., sous réserve que soit chaque fois vérifiée la capacité des peptides modifiés d'induire des anticorps susceptibles de neutraliser le poliovirus entier ou d'être reconnus par les anticorps monoclonaux CD-VP1. Il est naturellement entendu que tous ces équivalents possibles entrent dans le champ des revendications ci-après.

### PROPRIETES DES PEPTIDES SELON L'INVENTION.

Les peptides selon l'invention, et plus particulièrement les peptides conjugués formés,sont capables d'induire *in vivo* la production d'anticorps selon les techniques classiques. On peut les faire réagir avec les anticorps antipoliovirus. Ils induisent la synthèse d'anticorps antipoliovirus, lorsqu'ils sont inoculés à l'animal.

Par ailleurs on peut les utiliser comme réactifs pour le diagnostic et le titrage des anticorps antipoliomyélitiques. Dans leur utilisation comme réactifs pour un diagnostic, on peut avoir recours à des techniques classiques, par exemple à la technique ELISA. On rappelle ci-après le principe d'une telle méthode. Elle comprend, par exemple, les étapes suivantes :
- dépôt de quantités déterminées du peptide selon l'invention dans les puits d'un microplaque du type de celle utilisée pour la mise en oeuvre de la méthode ELISA ;
- introduction de dilutions croissantes du sérum contenant le cas échéant, les anticorps à détecter, voire à doser dans les puits de cette microplaque ;
- incubation et interruption de la réaction, par exemple par addition d'une solution d'acide sulfurique ;
- lavage poussé de la microplaque avec un tampon approprié ;
- introduction d' anticorps marqués dirigés contre les premiers, le marquage etant fait au moyen d'une enzyme capable d'hydrolyser un substrat choisi parmi ceux pour lesquels cette hydrolyse se manifeste par une variation d'absorbance pour une radiation de longueur d'onde donnée,
- mesure de la variation d'absorbance et
- détermination, de préférence vis-à-vis de mesures semblables faites vis-à-vis d'un témoin, de la teneur en anticorps du sérum étudié.

Les sequences d'ADN selon l'invention peuvent elles-mêmes être utilisées comme sondes d'hybridation permettant la détection de la présence d'ARN viral ou du cADN correspondant dans un échantillon biologique. Cette méthode impliquera par conséquence l'extraction préalable de l'ARN ou ADN de l'échantillon biologique et sa mise en contact dans des conditions permettant l'hybridation avec la séquence d'ADN selon l'invention marquée par un traceur radio-actif ou par une enzyme, notamment du genre de celles qui sont aptes à hydrolyser un substrat du type sus-indiqué.

L'invention concerne naturellement toutes les séquences d'ADN équivalentes conduisant à des produits d'expression doués de propriétés immunologiques équivalentes, en ce que les anticorps induits par les produits d'expression de ces séquences équivalentes sont à même de réagir avec les produits d'expression des fragments d'ADN plus particulièrement décrits et vice versa. En particulier, l'invention s'étend à des séquences d'ADN pouvant différer de celles qui ont été plus particulièrement décrites, par des délétions, additions ou substitutions d'acides nucléiques, pour autant que les propriétés immunologiques des produits d'expression soient équivalentes.

L'invention concerne encore un procédé d'obtention d'un peptide immunogène tel que ci-dessus défini comprenant les étapes que sont l'insertion d'une séquence d'ADN conforme à l'invention dans un vecteur approprié, la transformation d'un micro-organisme transformable par le vecteur ainsi modifié et capable d'exprimer la susdite séquence d'insertion, la récupération des protéines synthétisées et l'isolement de la fraction peptidique contenant le peptide selon l'invention, celui-ci pouvant être détecté, le cas échéant après un fractionnement en fonction des poids-moléculaires, par des anticorps actifs à la fois contre les particules "C" et "D" d'un même poliovirus et contre le poliopeptide structural VP-1 de la capside de ce poliovirus.

L'invention concerne naturellement également tout vecteur contenant une séquence d'insertion conforme à l'invention,sous le contrôle d'un promoteur permettant l'expression de cet insérat dans un micro-organisme transformable par ce vecteur.

Enfin l'invention concerne les micro-organismes transformés par un tel vecteur, aptes à produire une protéine reconnue par des anticorps actifs à la fois contre les particules "C" et "D" d'un même poliovirus et contre le polypeptide structural VP-1 de la capside de ce poliovirus.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes, notamment celles consistant en des séquences peptidiques correspondantes provenant d'autres souches de poliovirus, que ce soit de souches de type 1 ou encore de souches de types 2 ou 3. A titre d'exemple, on mentionnera les séquences correspondantes(ou équivalentes) de l'ADN codant pour la protéine VP1 de la souche Sabin. La séquence peptidique de la souche Sabin qui correspond à la séquence His 65 -Ala 106 de VP-1 dans la souche Mahoney, se distingue de celle-ci par des résidus aminoacyle distincts aux positions visées par les numéros indiqués ci-après :
- 88 (Ala), 90 (Ile), 95 (Ser), 98 (Lys) et 106 (Thr au lieu de Ala).
Il va de soi que les peptides qui comportent les différentes substitutions d'amino-acides qui ont été envisagées, constituent des équivalents de ceux plus sépcifiquement définis dans les revendications. Ces peptides sont donc, comme tels, également protégés par les revendications.

## Revendications

1. Fragment d'ADN caractérisé par une séquence nucléotidique codant pour tout ou partie de la séquence peptidique His 65-Phe 105 : 
His Val Val Gln His

Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 étant entendu qu'elle contient en tous les cas la séquence d'aminoacides :
Asp Asn Ser Ala Ser Thr Lys Asn Lys Asp Lys Leu.

2. Fragment selon la revendication 1, caractérisé en ce qu'il code pour une séquence :
Asp Asn Ser Ala Ser Thr Lys Asn Lys Asp Lys Leu.

3. Polypeptide contenant tout ou partie de la séquence d'aminoacides de formule : 
His Val Val Gln His
Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 ce polypeptide contenant en tous les cas la séquence :
Asp Asn Ser Ala Ser Thr Lys Asn Lys Asp Lys Leu.

4. Polypeptide selon la revendication 2, caractérisé par la séquence d'acides aminés de formule :
Asp Asn Ser Ala Ser Thr Lys Asn Lys Asp Lys Leu.

5. Polypeptide selon la revendication 3, caractérisé en ce qu'il contient moins de 20 résidus d'aminoacides.

6. Polypeptide selon la revendication 3, caractérisé en ce qu'il contient moins de 15 résidus d'aminoacides.

7. Conjugué immunogène résultant du couplage covalent d'un polypeptide selon l'une quelconque des revendications 3 à 6.

8. Vecteur contenant un insérat constitué par une séquence d'ADN placée sous le contrôle d'une promoteur permettant l'expression de cet insérat dans un micro-organisme transformable par ce vecteur, caractérisé en ce que la séquence de cet insérat nucléotidique est conforme à celle du fragment selon la revendication 1 ou la revendication 2.

9. Oligomère hydrosoluble, contenant notamment de 2 à 10 motifs monomères, caractérisé en ce que l'unité monomère consiste en un polypeptide tel que défini dans l'une quelconque des revendications 3 à 6.

10. Procédé de préparation d'un peptide tel que défini dans l'une quelconque des revendications 3 à 6 comprenant la transformation d'un micro-organisme transformable par le vecteur selon la revendication 8 et dans lequel le susdit insérat peut être exprimé sous le contrôle du promoteur correspondant, la récupération des protéines synthétisées et l'isolement de la fraction peptidique contenant le susdit peptide.

11. Réactif pour le diagnostic de la présence d'anticorps antipoliomyélitiques dans un échantillon biologique tel qu'un sérum, caractérisé en ce qu'il consiste en un antigène propre à réagir avec ces anticorps, cet antigène consistant en un polypeptide conforme à l'une quelconque des revendication 3 à 6 ou en un conjugué immunogène conforme à la revendication 7 ou encore en un oligomère immunogène conforme à la revendication 9.

12. Sonde pour la détection d'ARN du virus de la poliomyélite, mutant de la souche Sabin ou du cADN correspondant contenant une séquence nucléotidique capable de s'hybrider avec ledit ARN ou cADN, caractérisée en ce que cette sonde contient un fragment d'ADN selon la revendication 1 ou la revendication 2.

13. Utilisation d'un peptide selon l'une quelconque des revendications 3 à 6 d'un conjugué selon la revendication 7 ou d'un oligomère selon la revendication 9, pour la fabrication de principes de vaccins contre des poliovirus.

## Claims

1. DNA fragment characterized by a nucleotide sequence coding for all or part of the peptide sequence His 65 - Phe 105 : 
His Val Val Gln His

Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 it being understood that it contains in all cases the amino acid sequence :

Asp Asn Ser Ala Ser Thr Lys Asn Lys Asp Lys Leu.

2. Fragment according to Claim 1, characterized in that it codes for a sequence :

Asp Asn Ser Ala Ser Thr Lys Asn Lys Asp Lys Leu.

3. Polypeptide containing all or part of the amino acid sequence of formula: 
His Val Val Gln His

Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 this polypeptide containing in all cases the sequence :

Asp Asn Ser Ala Ser Thr Lys Asn Lys Asp Lys Leu.

4. Polypeptide according to Claim 2, characterized by the amino acid sequence of formula :

Asp Asn Ser Ala Ser Thr Lys Asn Lys Asp Lys Leu.

5. Polypeptide according to Claim 3, characterized in that it contains less than 20 amino acid residues.

6. Polypeptide according to Claim 3, characterized in that it contains less than 15 amino acid residues.

7. Immunogenic conjugate resulting from the covalent coupling of a polypeptide according to any one of the Claims 3 to 6.

8. Vector containing a insert constituted by a DNA sequence placed under the control of a promoter permitting the expression of this insert in a micro-organism which can be transformed by this vector, characterized in that the sequence of this nucleotide insert is in conformity with that of the fragment according to Claim 1 or Claim 2.

9. Water-soluble oligomer containing in particular 2 to 10 monomeric motifs, characterized in that the monomeric unit consists of a polypeptides such as defined in any one of the Claims 3 to 6.

10. Procedure for the preparation of a peptide such as that defined in any one of the Claims 3 to 6, comprising the transformation of a transformable micro-organism by the vector according to Claim 8 and in which the said insert can be expressed under the control of the corresponding promoter, the recovery of the proteins synthesized and the isolation of the peptide fraction containing the above-mentioned peptide.

11. Reagent for the diagnosis of the presence of anti-poliomyelitis antibodies in a biological sample such as serum, characterized in that it consists of a antigen capable of reacting with these antibodies, this antigen consisting of a polypeptide conforming to any one of the Claims 3 to 6 or of a immunogenic conjugate conforming to Claim 7 or also of an immunogenic oligomer conforming to Claim 9.

12. Probe for the detection of RNA of the poliomyelitis virus, mutant of the Sabin strain or of the corresponding cDNA containing a nucleotide sequence capable of hybridizing with the said RNA or cDNA, characterized in that this probe contains a DNA fragment according to Claim 1 or Claim 2.

13. Use of a peptide according to any one of the Claims 3 to 6, of a conjugate according to Claim 7 or a oligomer according to Claim 9, for the manufacture of vaccinating principles against polioviruses.

## Patentansprüche

1. DNA-Fragment, gekennzeichnet durch eine Nucleotidsequenz, die die Peptidsequenz His 65-Phe 105: 
His Val Val Gln His

Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 mit der Maßgabe, daß sie auf jeden Fall die Aminosäuresequenz Asp Asn Ser Ala Ser Thr Lys Asn Lys Asp Lys Leu enthält.

2. Fragment nach Anspruch 1, dadurch gekennzeichnet, daß es eine Sequenz Asp Asn Ser Ala Ser Thr Lys Asn Lys Asp Lys Leu codiert.

3. Polypeptid, das die Aminosäuresequenz der Formel: 
His Val Val Gln His

Arg Ser Arg Ser Glu Ser Ser Ile Glu Ser
70
Phe Phe Ala Arg Gly Ala Cys Val Ala Ile
80
Ile Thr Val Asp Asn Ser Ala Ser Thr Lys
90
Asn Lys Asp Lys Leu Phe Thr
100
 ganz oder teilweise enthält, wobei dieses Polypeptid auf jeden Fall die Sequenz Asp Asn Ser Ala Ser Thr Lys Asn Lys Asp Lys Leu enthält.

4. Polypeptid nach Anspruch 2, gekennzeichnet durch die Aminosäuresequenz der Formel Asp Asn Ser Ala Ser Thr Lys Asn Lys Asp Lys Leu.

5. Polypeptid nach Anspruch 3, dadurch gekennzeichnet, daß es weniger als 20 Aminosäurereste enthält.

6. Polypeptid nach Anspruch 3, dadurch gekennzeichnet, daß es weniger als 15 Aminosäurereste enthält.

7. Immunogenes Konjugat, das aus der kovalenten Kupplung eines Polypeptids nach einem der Ansprüche 3 bis 6 resultiert.

8. Vektor, der ein Insert enthält, das aus einer RNA-Sequenz besteht, die unter die Kontrolle eines Promotors gestellt ist, der die Expression dieses Inserts in einem durch diesen Vektor transformierbaren Mikroorganismus gestattet, dadurch gekennzeichnet, daß die Sequenz dieses Nucleotidinserts mit derjenigen des Fragmentes nach Anspruch 1 oder Anspruch 2 übereinstimmt.

9. Wasserlösliches Oligomeres, das insbesondere 2 bis 10 Monomereinheiten enthält, dadurch gekennzeichnet, daß die Monomereinheit aus einem Polypeptid, wie in einem der Ansprüche 3 bis 6 definiert, besteht.

10. Verfahren zur Herstellung eines Peptids, wie in einem der Ansprüche 3 bis 6 definiert, welches die Transformation eines Mikroorganismus, der durch den Vektor nach Anspruch 8 transformierbar ist und in welchem das oben genannte Insert unter Kontrolle des entsprechenden Promotors exprimiert werden kann, die Gewinnung der synthetisierten Proteine und die Isolierung der das genannte Peptid enthaltenden Peptidfraktion umfaßt.

11. Reagens für die Diagnose der Anwesenheit von Antipoliomyelitis-Antikörpern in einer biologischen Flüssigkeit, wie einem Serum, dadurch gekennzeichnet, daß es aus einem Antigen besteht, das mit diesen Antikörpern reagieren kann, wobei dieses Antigen aus einem Polypeptid nach einem der Ansprüche 3 bis 6 oder aus einem Immunogenkonjugat nach Anspruch 7 oder aus einem Immunogenoligomeren nach Anspruch 9 besteht.

12. Sonde für den Nachweis der RNA des Poliomyelitis-Virus, Mutante des Stammes Sabin, oder der entsprechenden cDNA, enthaltend eine Nucleotidsequenz, die in der Lage ist, sich mit der genannten RNA oder cDNA zu hybridisieren, dadurch gekennzeichnet, daß diese Sonde ein DNA-Fragment nach Anspruch 1 oder Anspruch 2 enthält.

13. Verwendung eines Peptids nach einem der Ansprüche 3 bis 6, eines Konjugats nach Anspruch 7 oder eines Oligomeren nach Anspruch 9 zur Herstellung von Impfstoffen gegen die Polioviren.
